(19) Europäisches Patentamt
European Patent Office
Office européen des brevets

(11) EP 0 398 293 B1

(12) EUROPEAN PATENT SPECIFICATION

(45) Date of publication and mention of the grant of the patent:
**13.08.1997 Bulletin 1997/33**

(51) Int Cl.⁶: **C07D 237/16**, C07D 401/12, A01N 43/58

(21) Application number: **90109243.7**

(22) Date of filing: **16.05.1990**

# (54) Pyridazinone derivatives and compositions for controlling and/or preventing insect pests

Pyridazinonderivate und Mittel zur Bekämpfung und/oder Vermeidung von Schaden durch Insekten

Dérivés de pyridazinone et compositions pour la lutte et/ou la prévention contre les insectes nuisibles

(84) Designated Contracting States:
**CH DE FR GB IT LI**

(30) Priority: **17.05.1989 JP 121603/89**
**28.12.1989 JP 343446/89**
**05.04.1990 JP 290926/90**

(43) Date of publication of application:
**22.11.1990 Bulletin 1990/47**

(73) Proprietor: **NISSAN CHEMICAL INDUSTRIES LTD.**
**Chiyoda-ku Tokyo (JP)**

(72) Inventors:
- **Ogura, Tomoyuki, c/o Nissan Chemical Ind., Ltd.**
  **Funabashi-shi, Chiba-ken (JP)**
- **Kawamura, Yasuo,**
  **c/o Nissan Chemical Ind., Ltd.**
  **Funabashi-shi, Chiba-ken (JP)**
- **Numata, Tatsuo, c/o Nissan Chemical Ind., Ltd.**
  **Funabashi-shi, Chiba-ken (JP)**
- **Umehara, Toshiyuki,**
  **c/o Nissan Chemical Ind., Ltd.**
  **Minami-saitama-gun, Saitama-ken (JP)**
- **Miyake, Toshiro, c/o Nissan Chemical Ind., Ltd.**
  **Minami-saitama-gun, Saitama-ken (JP)**
- **Haruyama, Hiroshi,**
  **c/o Nissan Chemical Ind., Ltd.**
  **Minami-saitama-gun, Saitama-ken (JP)**

(74) Representative:
**Hansen, Bernd, Dr. Dipl.-Chem. et al**
**Hoffmann Eitle,**
**Patent- und Rechtsanwälte,**
**Postfach 81 04 20**
**81904 München (DE)**

(56) References cited:

| | |
|---|---|
| EP-A- 0 088 384 | EP-A- 0 128 530 |
| EP-A- 0 183 212 | EP-A- 0 199 281 |
| EP-A- 0 210 647 | EP-A- 0 232 825 |
| EP-A- 0 283 271 | DE-A- 3 733 220 |

Remarks:
The file contains technical information submitted after the application was filed and not included in this specification

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 398 293 B1

## Description

The present invention relates to 3(2H)-pyridazinone derivatives and insecticidal, acaricidal and nematicidal compositions and compositions for expelling pests parasitic on animals containing as an active ingredient said derivatives.

The present invention concerns EP-A-0088384, EP-A-0134439, EP-A-0183212, EP-A-0199281, EP-A-0210647, EP-A-0193853, EP-A-0232825 and EP-A-0302346. The known compounds contained in these patent publications are represented by the following general formula (II):

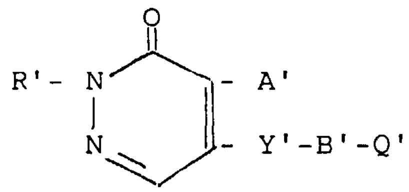

(II)

The characteristics of the compounds of these publications are, e.g., in the above formula (II): in case of EP-A-0088384, EP-A- 0134439, EP-A-0183212, EP-A-0199281 and EP-A-0232825, Y' represents oxygen atom or sulfur atom, but A' represents a substituent of such as halogen; in case of EP-A-0210647, R' represents an aryl group; in case of EF-A-0302346, R' represents a group such as alkyl group substituted by halogen atom. In EP-A-0193853, Y' represents nitrogen atom or oxygen atom, but A' represents a substituent of such as halogen. However, the compounds of the present invention are novel compounds which are not covered by these European patent publications.
German Patent Application DE-A-3733220 describes pyridazinone derivatives, a process for their production, a composition containing them and their use as pest control agents. These agents may contain mercapto and alkoxy derivatives attached to the pyridazinone ring.

European Patent Application EP-A-283271 reveals pyridazinone derivatives, a process for producing them and insecticides, acaricides and fungicides containing the derivatives. The ring system is substituted by a halogen atom and a substituted alkoxy or alkyl mercapto derivative.

An object of the present invention is to provide novel 3(2H)-pyridazinone derivatives which have insecticidal, acaricidal and nematicidal activities, and to provide a process for preparing these compounds.

Another object of the present invention is to provide insecticidal, acaricidal, nematicidal and molluscicidal compositions and compositions for expelling pests parasitic on animals, said compositions containing at least one of said derivatives as an active ingredient.

Still another object of the present invention is to provide a method for controlling and/or preventing insect pests by using the above-mentioned derivatives or compositions.

The present invention relates to 3(2H)-pyridazinone derivatives of the formula (I);

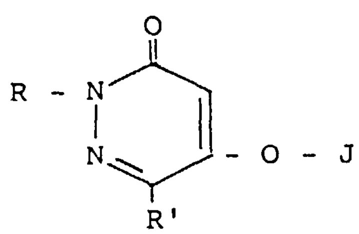

(I)

wherein,

R represents an alkyl group having 1 to 4 carbon atoms substituted by a phenyl group;

R' represents a hydrogen atom.

J represents

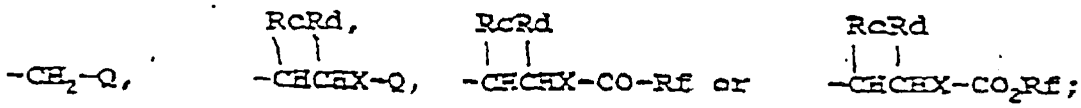

wherein:

Rc and Rd independently represent a hydrogen atom or an alkyl group having 1 to 4 carbon atoms, Rf represents a hydrogen atom, an alkyl group having 1 to 4 carbon atoms, an alkenyl group having 2 to 8 carbon atoms or a cycloalkyl group having 3 carbon atoms;
X represents - O - or - NH -; and
Q represents a pyridyl group or a pyridyl group substituted by a halogen atom.

The present inventors have found that the compounds of the general formula (I) have excellent insecticidal, acaricidal, nematicidal and molluscicidal activities.

For example, the known compounds of the formula (II) have strong insecticidal, acaricidal, nematicidal and fungicidal activities and have wide insecticidal spectrum and are excellent in prompt-effectiveness. On the other hand, the compounds of the present invention are slow in effectiveness because they have action to inhibit metamorphosis of insect pests. Moreover, the compounds of the present invention are effective, with very low drug-concentration, on various kinds of insect pests; e.g., agricultural insect pests such as green rice leaf hopper (Nephotettix cincticeps), brown rice planthopper (Nilaparvata lugens), green peach aphid (Myzus persicae), diamondback moth (Plutella xylostella), common cutworm (Spodoptera litura), two-spotted spider mite (Tetranychus urticae), citrus red mite (Panonychus citri), Kanzawa spider mite (Tetranychus kanzawai), sanitary insect pests such as house mosquito (Culex pipiens palens), housefly (Musca domestica), German cockroach (Blattella germanica), ant(Formicidae), chironomid (Chironomideae), flea (Siphonaptera), lice (Anoplura) ; stored product insect pests such as maize weevil (Sitophilus oryzae), red flour beetle (Tribolium castaneum), almond moth (Cadra cautella); house insect pests such as termites and veterinary insect pests such as ticks , acarids, fleas, lice, flies; house mites such as (Tyrophagus putrescentiae),(Dermatophagoides farinae), (Dermatophagoides pteronyssinus), (Cheyletus malaccensis); Mollusca such as slugs and snails and the like;

In other words, the compounds of the present invention can effectively control and prevent Dictyoptera, Isoptera, Hemiptera, Lepidoptera, Coleoptera, Hymenoptera, Diptera, ticks, acari and lice.

As the more preferable compounds, as being exemplified by the compound Nos. of Table 2 mentioned later, such as the following compounds may be mentioned. For example. Compound Nos. 5, 6, 8, 9, 31, 32, 33, 34, 57.

No.5

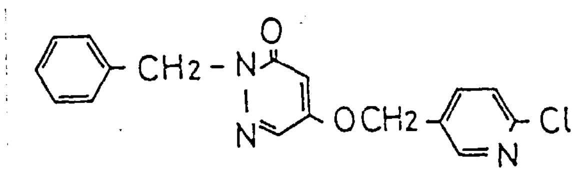

No. 6

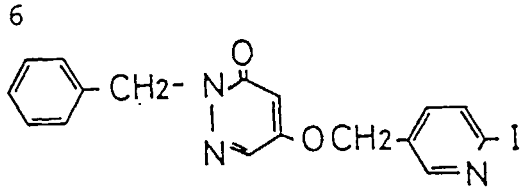

No. 8

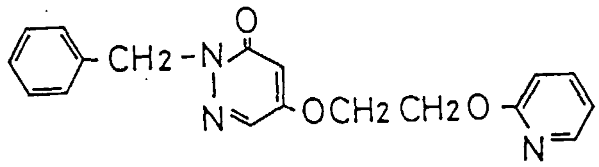

No. 31

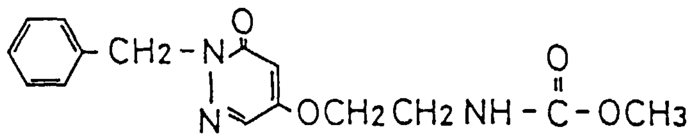

No. 32

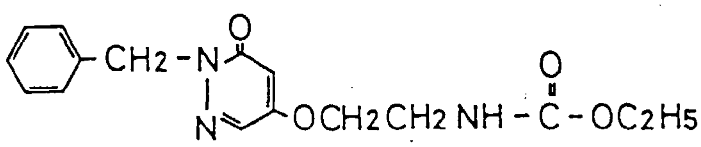

No. 33

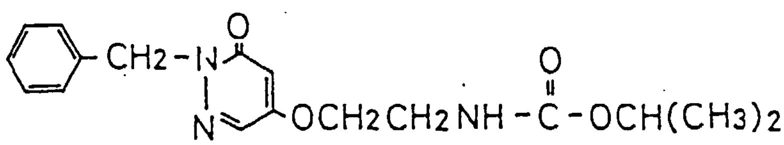

No. 57

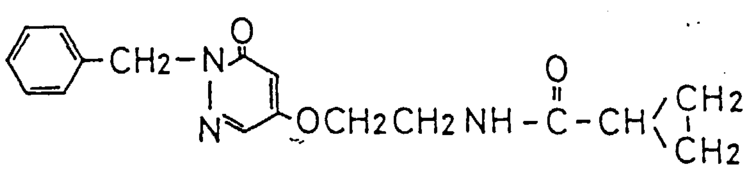

The compounds of the present invention may be produced by many producing methods. The methods are, for example, as follows:

Scheme (1)

Process 1-a

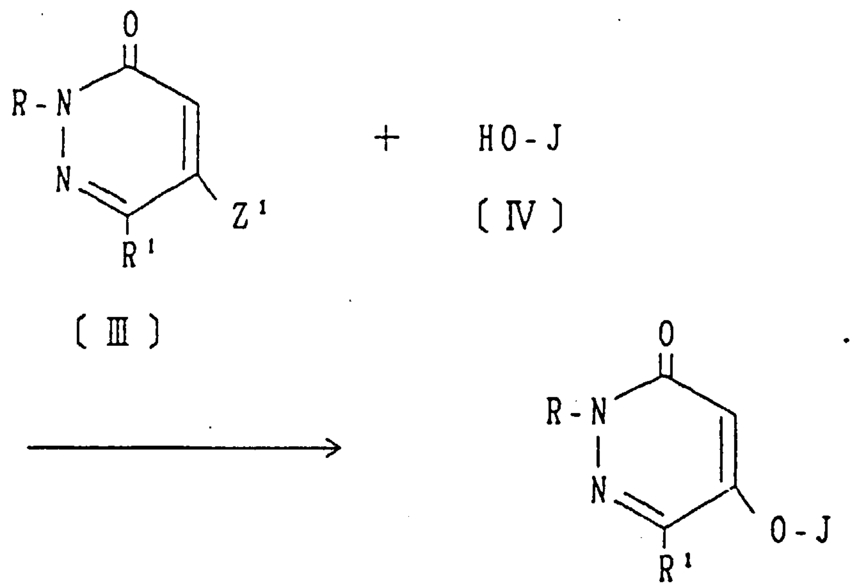

(IV)

(III)

Present compound (I)

Process 1-b

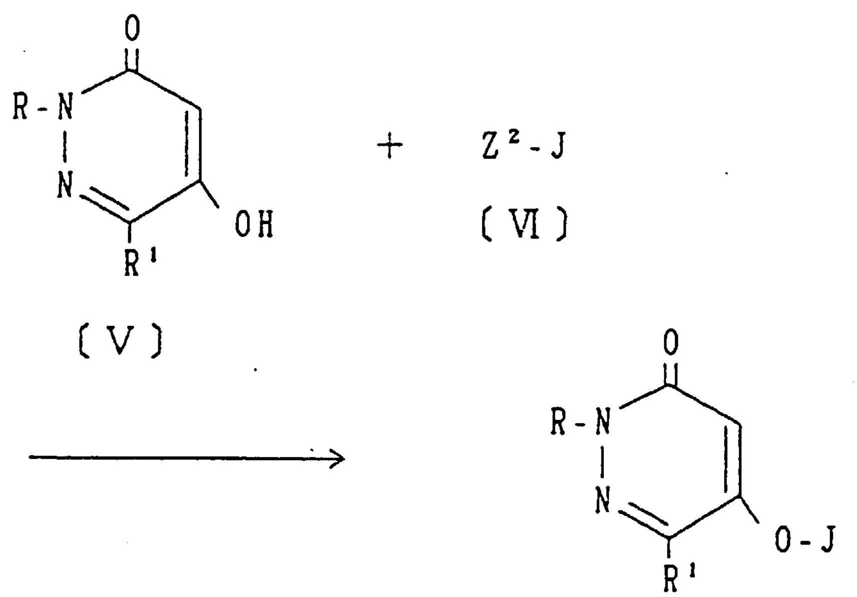

(VI)

(V)

Present compound (I)

Scheme (2)

Process 2 - a

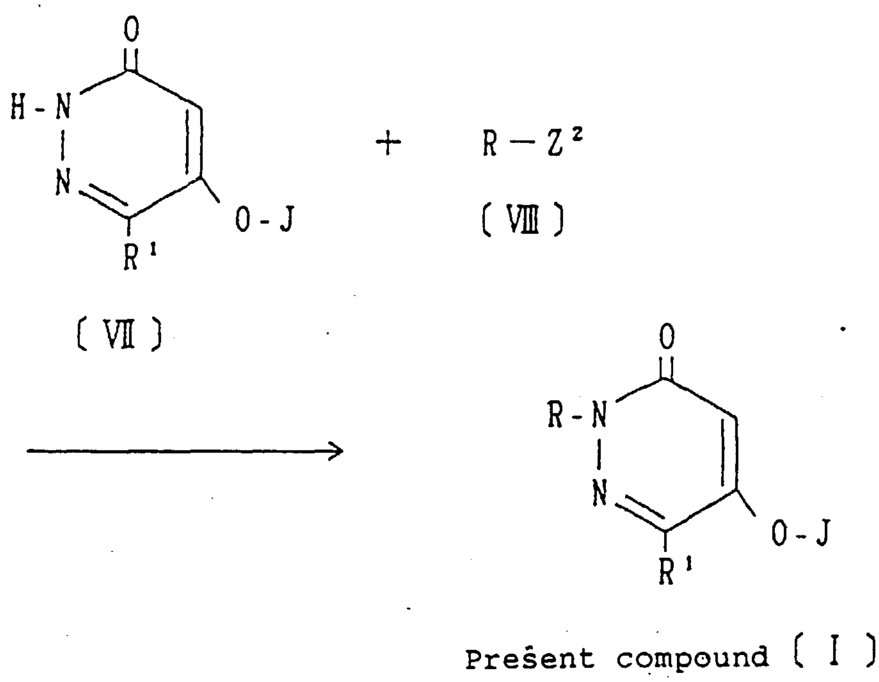

(VII)

$R - Z^2$

(VIII)

Present compound (I)

Process 2 - b

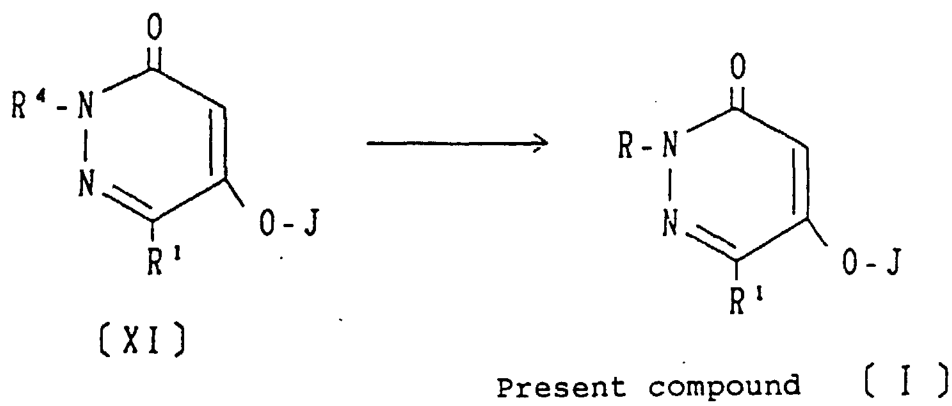

(XI)

Present compound (I)

In the above schemes (1) and (2) R, R', X, Rc, Rd, Q and J each have the same meanings as defined above and $Z^1$ represents halogen atom or azole group; $Z^2$ represents halogen atom, alkylsulfonate group or arylsulfonate group; $Z^3$ represents halogen atom, $R^4$ represents substituents having reactive functional group.

In the reaction shown in the schemes (1) and (2) as the solvent may be used lower alcohols such as methanol, ethanol; ketones such as acetone and methylethylketone; hydrocarbons such as benzene and toluene; ethers such as isopropyl ether, tetrahydrofuran and 1,4-dioxane; amides such as N,N-dimethylformamides and hexamethyl phosphoric triamide; halogenated hydrocarbons such as dichloromethane and dichloroethane. If necessary, these solvents may be used as a mixture or mixture with water.

As the base may be used inorganic bases such as sodium hydride, sodium hydroxide, potassium hydroxide, sodium carbonate, potassium carbonate, sodium hydrogencarbonate and organic bases such as sodium methoxide, sodium ethoxide, triethylamine or pyridine. If necessary, a tetraammonium salt such as triethylbenzylammonium chloride or the like can be added to reaction system as a catalyst. The reaction temperature ranges from -20°C to the boiling point

of the solvent used in the reaction system, and is preferably in the range of -5°C to the boiling point of the solvent used therein. Molar ratio of the starting materials can be optionally selected for reaction. However, it is advantageous to use the materials in an equimolar ratio or near such ratio.

More specifically, in the Process 1-a of the scheme (1), the compound of the present invention of the formula (I) can be produced by reacting Z' of the compound of the formula (III) with alcohols of the formula (IV) in a suitable solvent in the presence of the base. Z' is preferable to be halogen atom, especially, chlorine or bromine atom, and azoles, especially, 1-imidazole. As the solvent, it is preferable to use N,N-dimethylformamide, methanol, ethanol, toluene and a mixture solvent of toluene-water. As the base, it is preferable to use inorganic base, especially sodium carbonate, potassium carbonate, sodium hydroxide and potassium hydroxide. The reaction temperature is preferably in the range of from 20°C to 50°C.

In the Process 1-b, the present compounds can be produced by reacting pyridazinone derivatives of the formula (V) with alkyl halides or alkylsufonates of the formula (VI) in a suitable solvent in the presence of the base. $Z^2$ is preferable to be chlorine or bromine atom. As the solvent, it is preferable to use N,N-dimethylformamide, methanol, ethanol, acetonitrile, 1,2-dichloroethane, toluene and a mixture solvent of toluene-water. As the base, it is preferable to use inorganic base, especially, sodium carbonate, potassium carbonate, sodium hydroxide and potassium hydroxide. The reaction temperature is preferably in the range of from 20°C to 120°C. When sodium iodide, potassium iodide, tetrabutylammonium iodide are added in the reaction process, an excellent result may be obtained by the acceleration of the reaction.

In the Process 2 - a of the scheme (2), the present compounds can be produced by alkylating the 2-position of pyridazinone derivatives of the formula (VII) with R - $Z^2$ of the formula (VIII). In the above procedure, the present compounds may be readily produced by adding inorganic or organic bases to the reaction system to raise the reactivity of the pyridazinone derivatives of the formula (VII).

The Process 2 - b of the scheme (2) is a method to produce the present compound of the formula (I) by chemically modifying the functional groups in the N-substituent ($R^4$) of the pyridazinone derivatives of the formula (XI) to be converted into an intended N-substituent (R).

Concretely, a method in which halogen atom contained in $R^4$ is dehalogenated is mentioned.

As the method for preparing the compounds of the present invention, the following reactions are also useful.

Process 3

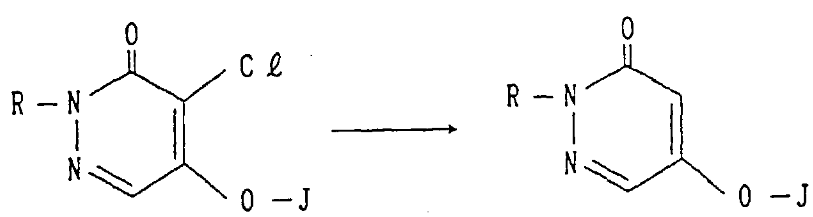

(In the Process 3 R and J represent the same meanings of the above)

In the Process 3, the compound of the present invention can be produced by dehalogenating the chlorin of 4-position of pyridazinone ring in a hydrogenating reaction. The application of this method is limited to the case where R and J are stable to the hydrogenating reaction.

The compounds encompassed by the present invention are illustrated in detail by the compounds listed in Table 1. However, it should be understood that the compounds in Table 1 are merely illustrated and not to restrict the present invention.

In the Table, Me represents methyl, Et represents ethyl, Pr represents propyl, Bu represents butyl, Ph represents unsubstituted phenyl, t represents tertiary, s represents secondary, i represents iso.

Incidentally, a compound of the present invention which contains asymmetric carbon atom(s) includes optically active (+) compound and (-) compound.

Furthermore, compounds among the present invention in which geometric isomer exists therein include cis compound and trans compound.

Table 1

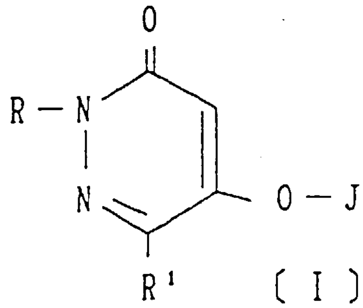

(I)

In the compound represented by the general formula (I),

| R | $R^1$ | J |
|---|---|---|
| $PhCH_2$ | H | $CH_2$(Q26-Cl-6) |
| $PhCH_2$ | H | $CH_2$(Q26-I-6) |
| $PhCH_2$ | H | $CH_2CH_2O$(Q17) |
| $PhCH_2$ | H | $CH_2CH$(Me)O(Q17) |
| $PhCH_2$ | H | $CH_2$(Q18) |

| R | $R^1$ | J |
|---|---|---|
| $PhCH_2$ | H | $CH_2CH_2OCOMe$ |
| $PhCH_2$ | H | $CH_2CH_2NHCOEt$ |
| $PhCH_2$ | H | $CH_2CH_2NHCO_2Me$ |
| $PhCH_2CH_2$ | H | $CH_2$(Q26-Cl-6) |
| $Ph_2CH$ | H | $CH_2$(Q26-I-6) |

| R | $R^1$ | J |
|---|---|---|
| $PhCH_2$ | H | $CH_2CH_2NHCO_2Et$ |
| $PhCH_2$ | H | $CH_2CH_2NHCO_2Pr$ |
| $PhCH_2$ | H | $CH_2CH_2NHCO_2Pr$-i |
| $PhCH_2$ | H | $CH_2CH_2NHCOPr$ |
| $PhCH_2$ | H | $CH_2CH_2NHCOC(Me)=CH_2$ |
| $PhCH_2$ | H | $CH_2CH_2NHCOCH=C(Me)_2$ |
| $PhCH_2$ | H | $CH_2CH_2NHCO$(Q1) |

| R | $R^1$ | J |
|---|---|---|
| $PhCH_2$ | H | $(CH_2)_3NHCO_2Me$ |
| $PhCH_2$ | H | $CH_2CH_2OCO$(Q1) |
| $PhCH_2$ | H | $(CH_2)_3COCO$(Q1) |
| $PhCH_2$ | H | $(CH_2)_3NHCO$(Q1) |
| $PhCH_2$ | H | $CH_2CH(Me)NHCO_2Et$ |
| $PhCH_2$ | H | $CH(Me)CH_2NHCO$(Q1) |

| R | $R^1$ | J |
|---|---|---|
| PhCH(Me) | H | $CH_2$(Q26-Cl-6) |
| PhCH(Me) | H | $CH_2CH_2O$(Q17) |
| PhCH(Me) | H | $CH_2CH_2NHCO_2Et$ |
| PhCH(Me) | H | $CH_2CH_2NHCO$(Q1) |
| $PhCH_2CH_2$ | H | $CH_2$(Q26-I-6) |
| $PhCH_2CH_2$ | H | $CH_2CH_2O$(Q17) |
| $PhCH_2CH_2$ | H | $CH_2CH_2NHCO_2Et$ |
| $PhCH_2CH_2$ | H | $CH_2CH_2NHCO$(Q1) |

| R | $R^1$ | J |
|---|---|---|
| $PhCH_2$ | H | $CH(Me)CH_2NHCO(Q1)$ |

Q1 Q17, Q18 and Q26 in Table 1 are the groups represented by the following formulae.

Q 1

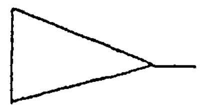

Q17

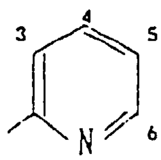

Q18

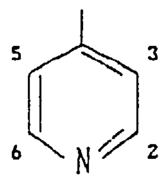

Q26

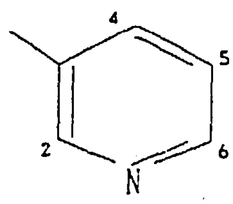

The preparations of the compounds of the present invention are described in detail by way of the following examples which are not to restrict the invention.

Preparation Example 1

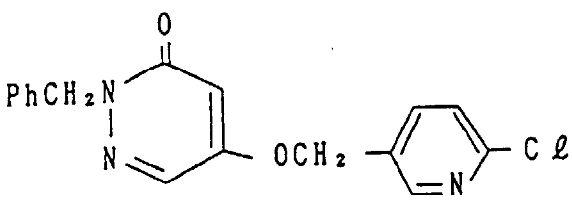

Synthesis of the compound No. 5 of the present invention.

In 20 ml of N,N-dimethylformamide were dissolved 1.2 g of 2-benzyl-5-hydroxy-3(2H)-pyridazinone and 1.3 g of 6-chloro-3-pyridinemethanolmesylate and 1.1 g of anhydrous potassium carbonate were added. The reaction mixture was heated and stirred for one hour in an oil bath of 70° C. After cooling to room temperature, the solution was poured into water, filtered off and washed with water. The crystals thus obtained were recrystalized from acetonitrile to give 760 mg of the intended compound.

melting point(m.p.): 161.7 - 162.5 °C

Preparation Example 2

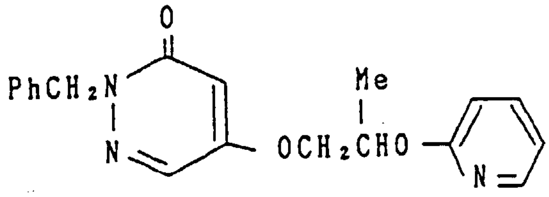

Synthesis of the compound No. 9 of the present invention

In 10 ml of N,N-dimethylformamide were dissolved 1.0 g of 2-benzyl-5-chloro-3(2H)-pyridazinone and 0.64 g of 2-(2-pyridyloxy)-1-propanol and were added 0.3 g of potassium hydroxide. The resulting solution was stirred for 24 hours at room temperature, then added with water to extract with ethyl acetate. The organic layer of the extract was washed with water, dried over anhydrous sodium sulfate and freed of ethyl acetate by distillation under reduced pressure to obtain a brown oil. This oil was purified by means of column chromatography (on silica gel, eluting with benzene-ethyl acetate 3/1) to give 300 mg of the intended compound.

melting point : 75.3 - 75.9 °C

Preparation Example 3

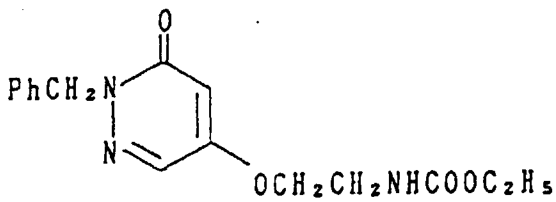

Synthesis of the compound No 32 of the present invention

In 20 ml of benzene were dissolved 0.6 g of 5-(2-aminoethyloxy)-2-benzyl-3(2H)-pyridazinone (Compound No. 30 of the present invention), and thereto were added 1 ml of triethylamine and 0.3 g of ethyl chloroformate under ice-cooling. The resulting mixture was stirred for two hours at room temperature, washed with aqueous sloution of diluted hydrochloric acid, and then the benzene layer thereof was washed with water, dried and freed of benzene by distillation to give 0.5 g of white crystals of the compound of the present invention.

melting point: 73.5 - 78.0 °C

Preparation Example 8

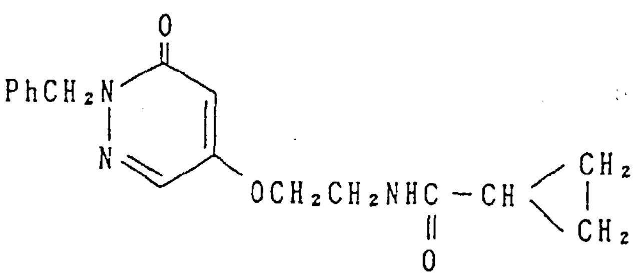

Synthesis of Compound No. 57 of the present invention

The mixture of 0.9 g of 5-(2-aminoethyloxy)-2-benzyl-3(2H)-pyridazinone (Compound No. 30 of the present invention), 0.4 g of triethylamine, 0.4 g of cyclopropanecarboxylic acid chloride and 30 ml of methylene chloride was heated and refluxed for 2 hours. After cooling to room temperature, the solution was washed with diluted aqueous hydrochloric acid solution and the layer of methylene chloride was dried and freed of the solvent to give raw crystals. The raw crystals thus obtained was purified by means of silica gel column chromatography (eluent: benzene/ethyl acetate 1/1)

to give 0.8 g of a white crystal of the compound of the present invention. melting point: 159.6 - 161.2 °C

Preparation Example 9

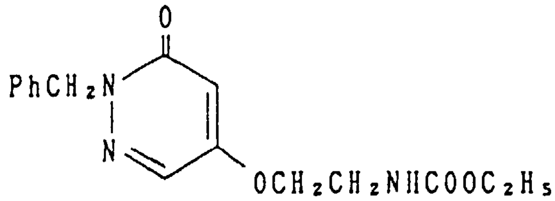

Synthesis of Compound No. 32 of the present invention

The mixture of 1.0 g of 2-benzyl-5-hydroxy-3(2H)-pyridazinone, 0.9 g of ethyl N-(2-chloroethyl) carbamate ($ClCH_2CH_2NHCO_2C_2H_5$), 1.4 g of anhydrous potassium carbonate and 30 ml of N,N-dimethylformamide was added with a catalytic amount of potassium iodide and heated for 7 hours at 70 - 80 °C. After the solvent was distilled off, the residue was added with water and extracted with chloroform. After drying the chloroform layer, raw crystals thus obtained by distillation was washed with the mixed solvent of isopropylether/benzene to give 0.9 g of a white crystal of the compound of the present invention.

Preparation Example 10

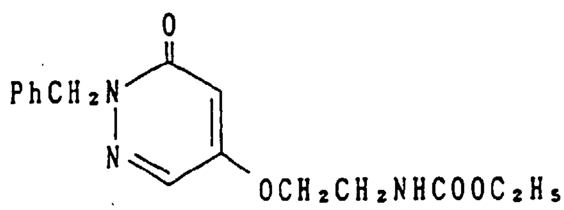

Synthesis of Compound No. 32 of the present invention

In 50 ml of ethanol were dissolved 1.0 g of 2-benzyl-4-chloro-5-(2-ethoxycarbonylamino)ethyloxy)-3(2H)-pyridazinone (white crystal: melting point 122.5 - 123.9 °C), and added with 5 % paradium-carbon catalyst (Pd/c). The solution was strongly stirred in the hydrogeneous atmosphere to carry out the reaction of dechlorination of 4 position. After a treatment, the reaction product thus obtained was repeated to be purified by a means of a thin layer chromatography to give 0.1 g of a white crystal of the compound of the present invention.

Reference Example 1

Synthesis of 2-benzyl-5-hydroxy-3(2H)-pyridazinone

To the mixture of 60 g of 2-benzyl-4,5-dichloro-3(2H)-pyridazinone and 38.8 g of potassium hydroxide were added with 250 ml of ethanol and 150 ml of water and heated in an oil bath to the refluxing temperature for 10 hours. After reaction the solvent was distilled off under reduced pressure, and to the residual solution was added with 200 ml of water and washed twice with chloroform, and water layer was acidified with concentrated hydrochloric acid. The white solid thus separated was filtered off, washed with water and dried to obtain 55 g of 2-benzyl-4-chloro-5-hydroxy-3(2H)-pyridazinone. Subsequently, in aqueous sodium hydroxide solution (NaOH 1.82 g, water 20 ml)were added 5.0 g of 2-benzyl-4-chloro-5-hydroxy-3(2H)-pyridazinone and 200 mg of 5 % Pd/C , then 474 ml of hydrogen gas were absorbed in the solution under normal pressure. After reaction, Pd/C was filtered off and the solution was added with concentrated hydrochloric acid to be acidified. The white solid thus separated was filtered off, washed with water and dried to obtain 4.1 g of 2-benzyl-5-hydroxy-3(2H)-pyridazinone.

melting point : 48 - 55 °C

Reference Example 2

Synthesis of 5-chloro-2-(cyclohexylmethyl)-3(2H)-pyridazinone

To 10.5 g of 2- (cyclohexylmethyl)-5-hydroxy-3(2H)-pyridazinone were added 25 ml of phosphorus oxychloride and heated for 4 hours at 85 °C. After reaction, the excess phosphorus oxychloride was distilled off under reduced pressure. The residual solution was poured with water and added with aqueous sodium hydroxide solution to be alkalified and the separated solid was extracted with benzene. The benzene layer was washed with water, dried over anhydrous sodium sulfate and filtered off with silica gel. Benzene was distilled off under reduced pressure to obtain 6.4 g of the intended compound.
melting point : 81.6 - 81.8 °C

Reference Example 3

Synthesis of 2-benzyl-5-(3-hydroxypropyloxy)-3(2H)-pyridazinone

The mixture of 10 g of 2-benzyl-5-chloro-3(2H)-pyridazinone, 50 g of 1,3-propanediol, 3.0 g of 85 % potassium hydroxide and 50 ml of N,N-dimethylformamide was reacted for 24 hours at room temperature, then the reaction solution was added with water and extracted with ethyl acetate, dried and freed of solvent to obtain 9.4 g of the intended compound.

a white colored crystal melting point: 91 - 93 °C

The physical properties of the compounds which were manufactured by the preparation methods illustrated in Preparation Examples 1 to 11 are shown in Table 2.

The compound Nos. of Table 2 will be referred to the ones of the Formulation Examples and Test Examples.

Table 2

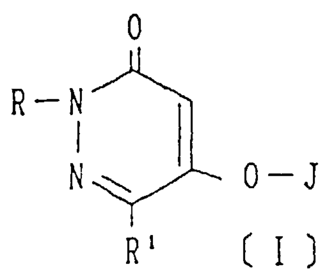

(I)

In the compound represented by the formula (I).

| No. | R | $R^1$ | J | m.p. (°C) |
|---|---|---|---|---|
| 5 | $PhCH_2$ | H | $CH_2$(Q26-Cl-6) | 161.7 - 162.5 |
| 6 | $PhCH_2$ | H | $CH_2$(Q26-I-6) | 173.6 - 174.8 |
| 8 | $PhCH_2$ | H | $CH_2CH_2O$(Q17) | 110.4 - 111.3 |
| 9 | $PhCH_2$ | H | $CH_2CH(Me)O$(Q17) | 75.3 - 75.9 |

| No. | R | $R^1$ | J | m.p. (°C) |
|---|---|---|---|---|
| 31 | $PhCH_2$ | H | $CH_2CH_2NHCO_2Me$ | 110.0-111.5 |
| 32 | $PhCH_2$ | H | $CH_2CH_2NHCO_2Et$ | 73.5-78.0 |
| 33 | $PhCH_2$ | H | $CH_2CH_2NHCO_2Pr$ | 77.0-79.5 |
| 34 | $PhCH_2$ | H | $CH_2CH_2NHCO_2Pr$-i | 114.9-117.3 |
| 37 | $PhCH_2$ | H | $CH_2CH_2NHCOPr$ | 113.0-118.0 |

| No. | R | $R^1$ | J | m.p. (°C) |
|---|---|---|---|---|
| 57 | $PhCH_2$ | H | $CH_2CH_2NHCO$(Q1) | 159.5-161.2 |
| 59 | $PhCH_2$ | H | $(CH_2)_3CNHCO_2Et$ | 112.4-113.1 |
| 60 | $PhCH_2$ | H | $(CH_2)_3CNHCO_2Me$ | 107.2-108.0 |

| No. | R | $R^1$ | J | m.p. (°C) |
|---|---|---|---|---|
| 61 | $PhCH_2$ | H | $CH_2CH_2OCO$(Q1) | 72.2- 73.5 |
| 62 | $PhCH_2$ | H | $(CH_2)_3COCO$(Q1) | 74.2- 75.4 |
| 70 | $PhCH_2$ | H | $(CH_2)_3CNHCO$(Q1) | 182.0-184.0 |
| 72 | $PhCH_2$ | H | $CH_2CH(Me)NHCO_2Et$ | |
| 73 | $PhCH_2$ | H | $CH(Me)CH_2NHCO$(Q1) | |

| No. | R | R' | J | m.p. (°C) |
|---|---|---|---|---|
| 109 | $PhCH_2$ | H | $CH_2$(Q17-$CF_3$-5) | |
| 111 | PhCH(Me) | H | $CH_2$(Q26-Cl-6) | |
| 112 | PhCH(Me) | H | $CH_2CH_2O$(Q17) | $N_D^{27}$ = 1.5846 |
| 113 | PhCH(Me) | H | $CH_2CH_2NHCO_2Et$ | 85 - 86 |
| 114 | PhCH(Me) | H | $CH_2CH_2NHCO$(Q1) | |
| 115 | $PhCH_2CH_2$ | H | $CH_2$(Q26-I-6) | 163.9 - 164.7 |
| 116 | $PhCH_2CH_2$ | H | $CH_2CH_2O$(Q17) | 117.9 - 119.4 |
| 117 | $PhCH_2CH_2$ | H | $CH_2CH_2NHCO_2Et$ | 85 - 87 |
| 118 | $PhCH_2CH_2$ | H | $CH_2CH_2NHCO$(Q1) | 162.6 - 163.2 |

| No. | R | R' | J | m.p. (°C) |
|---|---|---|---|---|
| 142 | PhCH(Me) | H | $CH_2$(Q26-I-6) | 124 - 127 |

When the compounds according to the present invention are used for compositions for preventing and controlling insect pests, they are generally mixed with suitable carriers, for instance, solid carriers such as clay, talc, bentonite or diatomaceous earth, or liquid carriers such as water, alcohols (e.g., methanol and ethanol), aromatic hydrocarbons (e. g., benzene, toluene and xylene), chlorinated hydrocarbons, ethers, ketones, esters (e.g., ethyl acetate) or acid amides (e.g., dimethylformamide). If desired, to these mixtures may be added emulsifier, dispersing agent, suspension agent, penetrating agent, spreader, stabilizer and the like to put them into practical use in the form of emulsifiable concentrate, oil solution, wettable powder, dust, granule, flowable or the like.

Moreover, the mixtures may be incorporated with other insecticides, various herbicides, fungicides, plant-growth regulating agents, synergists during formulation or application thereof, as necessary.

The amount of the compounds of the invention to be used as an active ingredient is suitably in the range of 0.005 to 50 kg per hectare although it varies depending upon the place and the seasons where the compounds are applied, manner of application, diseases and insect pests to be applied, cultivated crops to be protected and the like.

The following is formulation proportion and kinds of various preparations of the present invention.

| | Active ingredient | Carrier | Surface-active agent | Other component (adjuvant) |
|---|---|---|---|---|
| Emulsifiable concentrates | 1 - 25 | 52 - 95 | 3 - 20 | 0 - 20 |

(continued)

| | Active ingredient | Carrier | Surface-active agent | Other component (adjuvant) |
|---|---|---|---|---|
| Oil solutions | 1 - 30 | 70 - 99 | | |
| Flowables | 1 - 70 | 10 - 90 | 1 - 20 | 0 - 10 |
| Wettable powders | 1 - 70 | 15 - 93 | 3 - 10 | 0 - 5 |
| Dusts | 0.01 - 30 | 67 - 99.5 | | 0 - 3 |
| Granules | 0.01 - 30 | 67 - 99.5 | | 0 - 8 |
| Granule-shaped wettable powder | 1 - 90 | 5 - 90 | 1 - 50 | 0 - 30 |

The numeral values in the above table represent "percent by weight".

In use, emulsifiable concentrates, oil solutions, flowables, wettable powder and granule-shaped wettable powder are diluted with a predetermined amount of water and applied. Dusts and granules are directly applied without being diluted with water. The granules contain baits.

Each component of the above formulations is exemplified as follows.

Emulsifiable concentrates

| | |
|---|---|
| Active ingredient: | Present compound |
| Carrier: | xylene, dimethylformamide, methylnaphthalene, cyclohexanone, dichlorobenzene, isophorone |
| Surface-active agent: | Sorpol 2680, Sorpol 3005X, Sorpol 3353 |
| Other ingredients: | piperonylbutoxide, benzotriazole |

Oil solution

| | |
|---|---|
| Active ingredient: | Present compound |
| Carrier: | xylene, methylcellosolve, kerosene |

Flowables

| | |
|---|---|
| Active ingredient: | Present compound |
| Carrier: | water |
| Surface-active agent: | Lunox 1000C, Sorpol 3353, Soprophor FL, Nippol, Agrisol S-710, sodium lignin sulfonate |
| Other ingredients: | Xanthan gum, formalin, ethylene glycol, propylene glycol |

Wettable powders

| | |
|---|---|
| Active ingredient: | Present compound |
| Carrier: | calcium carbonate, kaolinite, Zeeklite D, Zeeklite PFP, diatomaceous earth, talc |
| Surface-active agent: | Sorpol 5039, Lunox 1000C, calcium lignin sulfonate, sodium dodecyl benzenesulfonate, Sorpol 5050, Sorpol 005D, Sorpol 5029-0 |
| Other ingredient: | Carplex #80 |

Dusts

| | |
|---|---|
| Active ingredient: | Present compound |
| Carrier: | calcium carbonate, kaolinite, Zeeklite D, talc |
| Other ingredient: | diisopropyl phosphate, Carplex #80 |

Granules (1)

| | |
|---|---|
| Active ingredient: | Present compound |
| Carrier: | calcium carbonate, kaolinite, bentonite, talc |
| Other ingredients: | calcium lignin sulfonate, polyvinyl alcohol |

Granules (2) (Bait)

| | |
|---|---|
| Active ingredient: | Present compound |
| Carrier: | wheat flour, wheat bran, corn grits, Zeeklite D |
| Other ingredients: | paraffin, bean oil |

Granule-shaped wettable powder

| | |
|---|---|
| Active ingredient: | Present compound |
| Carrier: | calcium carbonate, kaolinite, Zeeklite, clay, ammonium sulfate, urea, white carbon |
| Surface-active agent: | Lunox 1000C, formarin-condensation agent of naphthalenesulfonate, polyoxyethylene-nonylphenylether, sodium lignin sulfonate |
| Other ingredient: | stabilizer such as epoxidized bean oil, anti-oxidatnt |

In the following, there are shown formulation examples of compositions for preventing and controlling insect pests, said compositions containing the compounds of the present invention as an active ingredient. These examples are only illustrative and not to restrict the present invention. In the following formulation examples, "part" means "part by weight".

Formulation Example 1: Emulsifiable concentrate

| | |
|---|---|
| Present compound: | ... 5 part |
| Xylene | ... 70 parts |
| N,N-dimethylformamide | ... 20 parts |
| Sorpol 2680 (trade name: a mixture of a non-ionic surface-active agent and an anionic surface-active agent manufactured by Toho Chemical, Co., Ltd., Japan) | ... 5 parts |

In the above respective emulsifiable concentrates, each component of the respective emulsifiable concentrates are mixed intimately to form respective emulsifiable concentrates. Upon use, the emulsifiable concentrates are diluted with water up to one fiftieth to one twenty thousandth in concentration and applied at a rate of 0.005 to 50 kg of the active ingredient per hectare.

Formulation Example 3: Oil solutions

| | |
|---|---|
| Present compound | 10 part |
| Methylcellosolve | 90 parts |

In the above respective oil solutions, each component of the respective oil solutions are homogeneously mixed together to form the respective oil solutions. Upon use, the oil solutions are applied at a rate of 0.005 to 50 kg of the active ingredient per hectare.

Formulation Example 5: Granules

| | |
|---|---|
| Present compound | 5 part |
| Bentonite | 54 parts |

(continued)

| | |
|---|---|
| Talc | 40 parts |
| Calcium lignin sulfonate | 1 part |

In the above granules, components of granules are mixed intimately together and ground, incorporated with a small amount of water and mixed together with stirring. The resulting mixture is granulated by means of extrusion-granulator and dried to form granules. Upon use, the granule is applied to at a rate of 0.005 to 50 kg of the active ingredient per hectare.

Formulation Example 6: Flowables

| | |
|---|---|
| Present compound | 35 part |
| Sorpol 3353 (trade name, non-ionic surface-active agent manufactured by Toho Chemicals, Co., Ltd., Japan) | 10 part |
| Lunox 1000C (trade name, as anionic surface-active agent manufactured by Toho Chemicals, Co., Ltd., Japan) | 0.5 part |
| 1% aqueous solution of Xanthan gum (natural high-molecular compound) | 20 parts |
| Water | 34.5 parts |

In the above respective flowables, each component except the active ingredient (present compound) are uniformly mixed together to form a solution. The resulting mixture is added by the present compound, thoroughly stirred and wet-ground by means of sand mill to form respective flowables. Upon use, the flowables are diluted up to one fiftieth to one twenty thousandth with water and applied at a rate of 0.005 to 50 kg of the active ingredient per hectare.

Formulation Example 7: Granule-shaped wettable powder

| | |
|---|---|
| Present compound | 50 parts |
| Clay | 10 parts |
| Ammonium sulfate | 20 parts |
| Sodium lignin sulfonate | 10 parts |
| Formalin-condensation product of naphthalene sulfonate | 10 parts |

The components are uniformly mixed and ground and kneadered with water and then, subjected to an extruding granulator having a screen of 0.5 mm ϕ to be granulated. The water of the granuled product is dried to produce granule-shaped wettable powder. Upon use, the granule-shaped wettable powder is applied at a rate of 0.005 to 50 kg of the active ingredient per hectare.

In the following, the effects of the present compounds as an insecticide are explained in detail by way of the test examples.

Test Example 1: Insecticidal test on Green rice leafhopper (Nephotettix cincticeps)

A 5% emulsifiable concentrate (or a 25% wettable powder) of a compound of the present invention was diluted with water containing a spreader to give a 500 ppm solution of the compound.

The stems and leaves of rice-plant in a 1/20000 are pot were sufficiently applied with the resulting solution and then air-dried. Thereafter, 20 second instar nymphae of green rice leafhopper (Nephotettix cincticeps) which resist organic phosphorous insecticides and carbamate insecticides were released in the pot.

The rice-plant thus treated was covered with a cylindrical wire gauze and kept in a thermostatic chamber.

Thirty (30) days after, the number of the green rice leafhoppers parasitic on the rice-plant was counted and the mortality thereof was determined according to the following equation:

$$\text{Mortality (\%)} = \frac{\text{number of the insect killed}}{\text{number of insect released}} \times 100$$

The test was conducted twice for each compound. In the results, the following compounds exhibited high effects of 100% of mortality.

Compound Nos. 5, 6, 31, 33,

Test Example 2: Insecticidal test on Brown rice planthopper (Nilaparvata lugens)

The procedures in Test Example 1 were repeated by using second instar nymphae of brown rice planthopper (Nilaparvata lugens) instead of the second instar nymphae of green rice leafhoppers which resist organic phosphorous insecticides and carbamate insecticides. In the results, the following compounds exhibited high effects of 100% of mortality.

Compound Nos. 5, 6, 31, 32, 33, 34, 57, 59, 61,

Test Example 3: Insecticidal test on Red flour beetle (Tribolium castaneum)

In a transparent small test tube was placed 5% emulsifiable concentrate of a compound of the present invention (or a 25% wettable powder or a 20% oil solution thereof), and thereto was added acetone to give a 500 ppm acetone solution of the compound. Ten (10) cc of the acetone solution was added to 10 g of wheat flour placed in a laboratory dish of 9 cm in diameter. After stirring, acetone was distilled away from the mixture. Then, 10 adults each of male and female red flour beetle (Tribolium castaneum) were released in the dish. The dish containing the adults was kept in a thermostatic chamber.

Ninety (90) days after, evaluation was conducted by counting the number of the adults which came out.

The test was conducted twice of each compound.

As a result, no emerged adult was observed at all in the dish treated with any one of the following compounds.

Compound Nos. 5, 6, 8, 9, 31, 32, 33, 34, 57,

Test Example 4: Insecticidal test on House mosquito (Culex pipiens pallens)

A 5% emulsifiable concentrate (or a 25% wettable powder or 20% oil solution) of a compound of the present invention was diluted with deionized water to give a 10 ppm solution of the compound.

Two hundred (200) ml of the solution was poured in a tall laboratory dish of 9 cm in diameter and 6 cm in height. Ten larvae of house mosquito (Culex pipiens pallens) were repeased in the dish. The dish containing the larvae was kept in a thermostatic chamber of 25 °C.

Seven (7) days after, the number of the larvae killed was counted.

The test was conducted twice of each compound.

As the result, no emerged adult was observed at all in the dish treated with any one the following compounds.

Compound Nos. 5, 6, 8, 9, 57,

Test Example 5: Insecticidal test on Almond moth (Cadra cautella)

In a transparent small test tube was placed 5% emulsifiable concentrate of a compound of the present invention (or a 25% wettable powder or a 20% oil solution thereof), and thereto was added acetone to give a 500 ppm acetone solution of the compound. Ten (10) cc of the acetone solution was added to 10 g of rice bran placed in a laboratory dish of 9 cm in diameter. After stirring, acetone was distilled away from the mixture. Then, 10 larvae of almond moth (Cadra cautella) were released in the dish. The dish containing the larvae was kept in a thermostatic chamber.

Thirty (30) days after, evaluation was conducted by counting the number of the adults which came out.

The test was conducted twice of each compound.

As a result, no emerged adult was observed at all in the dish treated with any one of the following compounds.

Compound Nos. 5, 6, 8 , 9, 31, 32, 33, 37, 57,

Test Example 6: Insecticidal test on Diamond back moth (Plutella xylostella)

A 5% emulsifiable concentrate (or a 25% wettable powder) of a compound of the present invention was diluted with water containing a spreader to give a 500 ppm solution of the compound.

Leaves of cabbage (Brassica oleracea) were dipped in the solution, air-dried and then put in a laboratory dish of 7 cm in diameter. Ten (10) third instar larvae of diamond back moth (Plutella xylostella) were released in each laboratory dish and kept in a thermostatic chamber.

Twenty (20) days after, the number of emerged adults was counted and the mortality thereof was determined according to the equation as described in Test Example 1. The test was conducted twice of each compound.

As a result, the follwoing compounds exhibited high effects of 100% of mortality.
Compound Nos. 5, 6, 9, 31, 32, 33, 34, 37, 57,

Test Example 7: Insecticidal test on Maize weevil (Sitophilus oryzae)

A 5% emulsifiable concentrate (or a 25% wettable powder) of a compound of the present invention was diluted with water containing a spreader to give a 500 ppm solution of the compound.

Ten (10) g of unmilled rice in a laboratory dish were dipped with the obtained solution and air-dried, and then maize weevil adults each 10 of male and female were released therein. The laboratory dishes were kept in a thermostatic chamber.

Ninety (90) days after, evaluation was conducted by counting the number of the adults which came out.

The test was conducted twice of each compound.

As a result, no emerged adult was observed at all in the dishes treated with any one of the follwoing compounds.
Compound Nos. 5, 6, 8, 9, 31, 32, 34, 57,

Test Example 8: Insecticidal test on German cockroach (Blattella germanica)

In a transparent small test tube was weighed and placed 5% emulsifiable concentrate of a compound of the present invention (or a 25% wettable powder or a 20% oil solution thereof), and theretb was added acetone to give a 500 ppm acetone solution of the compound. Ten (10) cc of the acetone solution was added to 10 g of powder feed for small animals placed in a laboratory dish of 9 cm in diameter. After stirring, acetone was distilled away from the mixture. This laboratory dish was placed in a large laboratory dish of 20 cm in diameter to prepare a bait. In this large laboratory dish were released 10 five instar nymphae of German cockroaches (Blattella germanica). The large laboratory dish was kept in a thermostatic chamber. In the large laboratory dish, a laboratory dish containing moistured sanitary cotton was placed so as to give water to the nymphae.

Sixty (60) days after, evaluation was conducted by counting the number of the adults which came out.

The test was conducted twice of each compound.

As a result, no adult was observed at all in the dish treated with any one of the following compounds.
Compound Nos. 5, 6, 8, 9, 31, 32, 33, 34, 37, 57

Test Example 9: Insecticidal test on Housefly (Musca domestica)

A 5% emulsifiable concentrate (or a 25% wettable powder) of a compound of the present invention was diluted with water to give a 500 ppm solution of the compound.

One (1) cc of the solution was dropwise applied on a filter paper placed in a laboratory dish of 9 cm in diameter. Ten larvae of housefly (Musca domestica) were released in the dish. The dish containing the larvae was kept in a thermostatic chamber of 25 °C.

Two (2) weeks after, the number of the larvae killed was counted.

The test was conducted twice of each compound.

As the result, no emerged adult was observed at all in the dish treated with any one the following compounds.
Compound Nos. 8, 9, 57,

## Claims

### Claims for the following Contracting States : CH, DE, FR, GB, IT, LI

**1.** A pyridazinone derivative of the formula (I)

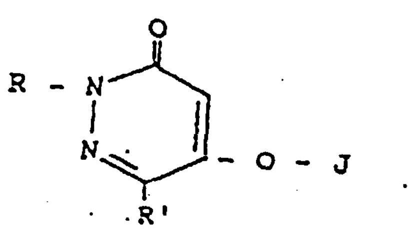

(I)

wherein R represents an alkyl group having 1 to 4 carbon atoms substituted by a phenyl group,

R' represents a hydrogen atom,
J represents

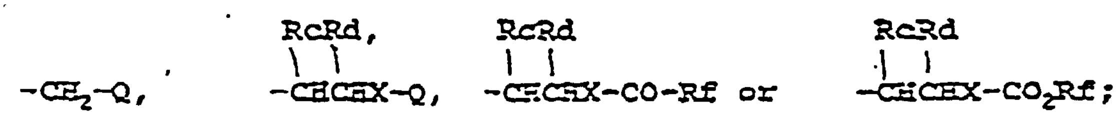
or

wherein:

Rc and Rd independently represent a hydrogen atom or an alkyl group having 1 to 4 carbon atoms, Rf represents a hydrogen atom, an alkyl group having 1 to 4 carbon atoms, an alkenyl group having 2 to 8 carbon atoms or a cycloalkyl group having 3 carbon atoms;
X represents - O - or - NH -; and
Q represents a pyridyl group or a pyridyl group substituted by a halogen atom.

**2.** A pyridazinone derivative of the formula (I) according to claim 1

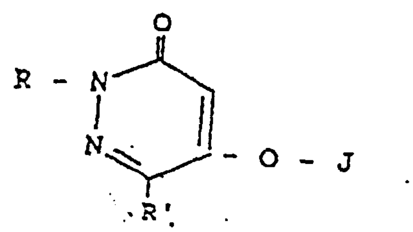
(I )

wherein R represents an alkyl group having 1 carbon atom substituted by a phenyl group,

R' represents a hydrogen atom,
J represents

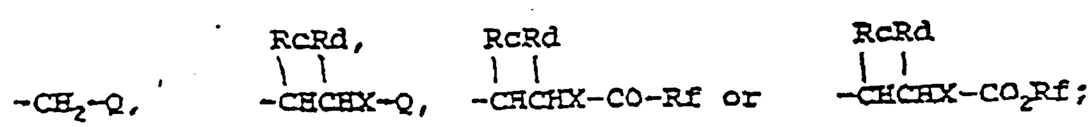
or

wherein:

Rc and Rd independently represent a hydrogen atom or a methyl group, Rf represents a hydrogen atom, an alkyl group having 1 to 4 carbon atoms, an alkenyl group having 2 to 8 carbon atoms or a cycloalkyl group having 3 carbon atoms;
X represents - O - or - NH -; and
Q represents a pyridyl group or a pyridyl group substituted by a halogen atom.

**3.** A pyridazinone derivative according to Claim 1 comprising the following compounds :

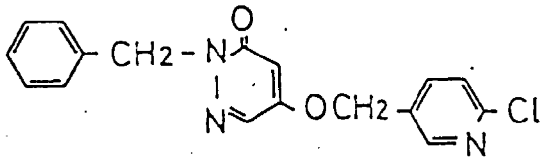

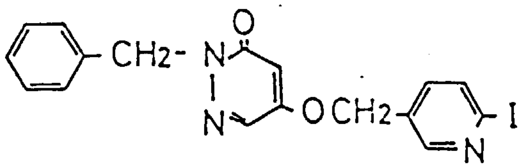

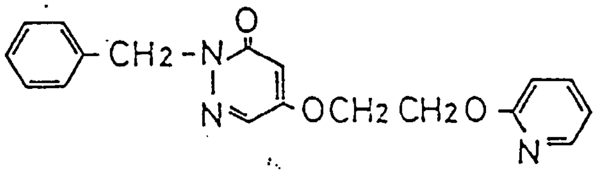

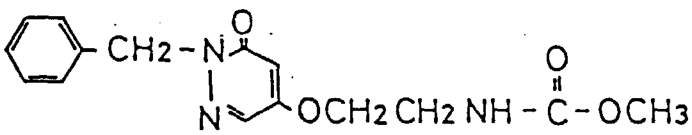

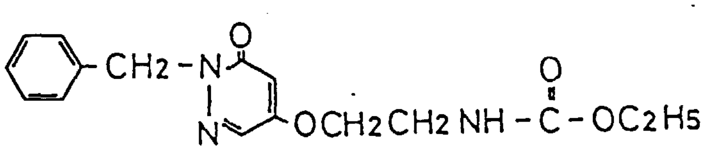

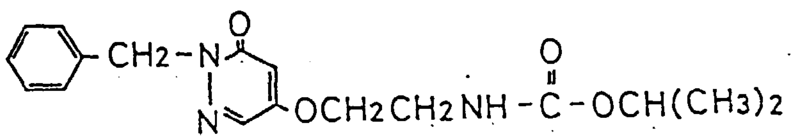

and

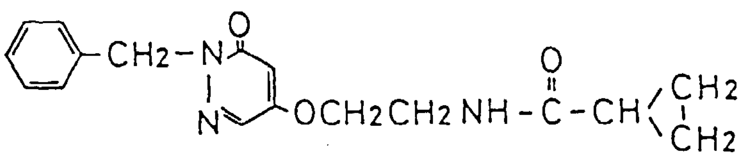

4. A process for the preparation of pyridazinone derivatives as defined in claim 1 by reacting the pyridazinone compound of the general formula (IB)

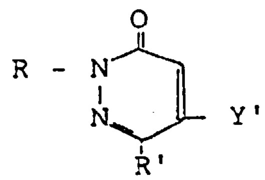

(IB)

with the compound of the general formula (IC)

$$Y^2 - J \quad (IC);$$

(wherein R, R' and J independently represent the meaning as in Claim 1 or 2, Y' and $Y^2$ independently represent halogen atom or OM group (wherein M represents hydrogen atom or alkaline metal), with the proviso that when Y' represents halogen atom $Y^2$ represents OM group, and when Y' represents OM group $Y^2$ represents halogen atom.).

**5.** Compositions for controlling and/or preventing pests containing as an active ingredient one or more of the 3(2H)-pyridazinone derivatives as defined in claim 1.

**6.** A non-therapeutical method for controlling and/or preventing pests at a locus which comprises applying to said locus a pesticidally amount of one or more of the 3(2H)-pyridazinone derivatives as defined in claim 1, together with a carrier if necessary.

**7.** A non-therapeutical method according to claim 6, wherein the pests to be controlled are insects and representatives of the order Acarina.

**8.** A non-therapeutical use of a pyridazinone derivatives as defined in claim 1, 2 or 3 for controlling and/or preventing pests.

**9.** 2-benzyl-5-hydroxy-3(2H)-pyridazinone.

## Patentansprüche

### Patentansprüche für folgende Vertragsstaaten : CH, DE, FR, GB, IT, LI

**1.** Pyridazinon-Derivat der Formel (I)

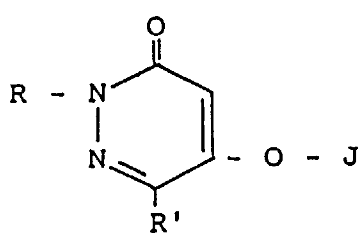

(I)

in der R eine Alkyl-Gruppe mit 1 bis 4 Kohlenstoffatomen darstellt, die durch eine Phenyl-Gruppe substituiert ist;

R' ein Wasserstoffatom darstellt;

J $-CH_2-Q$,

```
RcRd,        RcRd                     RcRd
| |          | |                      | |
-CHCHX-Q,    -CHCHX-CO-Rf     oder    -CHCHX-CO2Rf
```

darstellt, in denen

Rc und Rd unabhängig voneinander ein Wasserstoffatom oder eine Alkyl-Gruppe mit 1 bis 4 Kohlenstoffatomen darstellen; Rf ein Wasserstoffatom, eine Alkyl-Gruppe mit 1 bis 4 Kohlenstoffatomen, eine Alkenyl-Gruppe mit 2 bis 8 Kohlenstoffatomen oder eine Cycloalkyl-Gruppe mit 3 Kohlenstoffatomen darstellt;

X -O- oder -NH- darstellt; und

Q eine Pyridyl-Gruppe oder eine durch ein Halogenatom substituierte Pyridyl-Gruppe darstellt.

**2.** Pyridazinon-Derivat der Formel (I) nach Anspruch 1

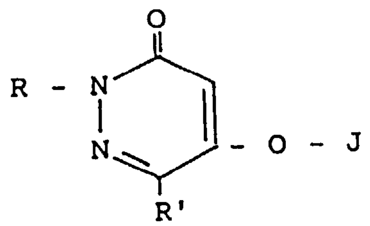

(I)

in der R eine Alkyl-Gruppe mit 1 Kohlenstoffatom, die durch eine Phenyl-Gruppe substituiert ist, darstellt;

R' ein Wasserstoffatom darstellt;

J $-CH_2$-Q,

```
RcRd,        RcRd                     RcRd
| |          | |                      | |
-CHCHX-Q,    -CHCHX-CO-Rf     oder    -CHCHX-CO2Rf
```

darstellt, in denen

Rc und Rd unabhängig voneinander ein Wasserstoffatom oder eine Methyl-Gruppe darstellen; Rf ein Wasserstoffatom, eine Alkyl-Gruppe mit 1 bis 4 Kohlenstoffatomen, eine Alkenyl-Gruppe mit 2 bis 8 Kohlenstoffatomen oder eine Cycloalkyl-Gruppe mit 3 Kohlenstoffatomen darstellt;

X -O- oder -NH- darstellt; und

Q eine Pyridyl-Gruppe oder eine mit einem Halogenatom substituierte Pyridyl-Gruppe darstellt.

**3.** Pyridazinon-Derivat nach Anspruch 1, das die folgenden Verbindungen umfaßt:

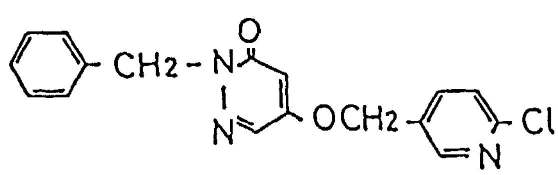

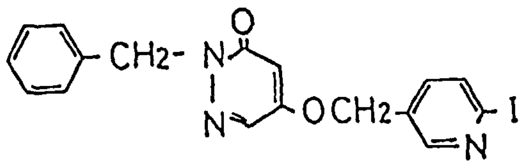

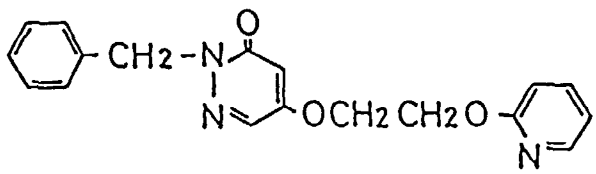

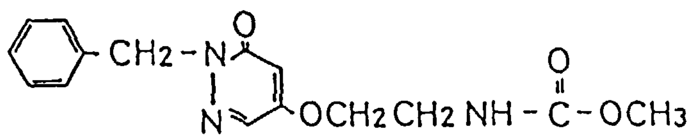

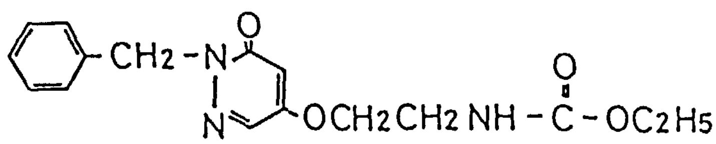

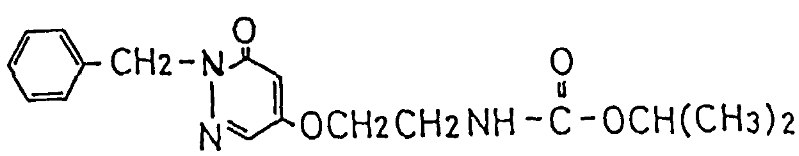

und

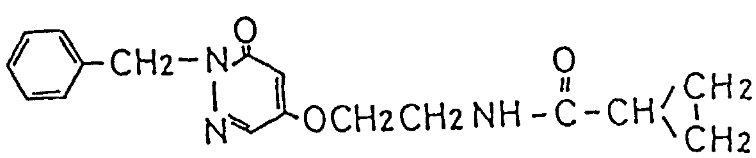

4. Verfahren zur Herstellung von Pyridazinon-Derivaten, wie sie in Anspruch 1 definiert sind, in dem die Pyridazinon-Verbindung der allgemeinen Formel (IB)

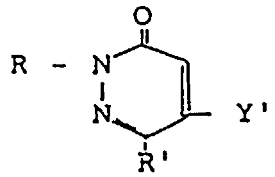

(IB)

mit der Verbindung der allgemeinen Formel (IC)

$$Y^2\text{-}J \qquad (IC)$$

umgesetzt wird (dabei stellen R, R', und J unabhängig voneinander dieselben Bedeutungen wie in Anspruch 1 oder 2 dar, stellt Y' und stellt $Y^2$ unabhängig ein Halogenatom oder eine OM-Gruppe dar (worin M ein Wasserstoffatom oder ein Alkalimetall ist), unter der Voraussetzung, daß wenn Y' ein Halogenatom darstellt, $Y^2$ eine OM-Gruppe ist, und wenn Y' eine OM-Gruppe darstellt, $Y^2$ ein Halogenatom ist).

**5.** Zusammensetzungen zur Bekämpfung von und/oder zum Schutz vor Schädlingen, die als aktives Ingrediens eines oder mehrere der 3(2H)-Pyridazinon-Derivate, wie sie in Anspruch 1 definiert sind, enthalten.

**6.** Nicht-therapeutisches Verfahren zur Bekämpfung von und/oder zum Schutz vor Schädlingen an einem Ort, das ein Aufbringen einer pestiziden Menge eines oder mehrerer 3(2H)-Pyridazinon-Derivate, wie sie in Anspruch 1 definiert sind, wenn notwendig, zusammen mit einem Träger auf diesen Ort umfaßt.

**7.** Nicht-therapeutisches Verfahren nach Anspruch 6, in dem die zu bekämpfnden Schädlinge Insekten und Vertreter der Ordnung Acaria sind.

**8.** Nicht-therapeutische Verwendung von Pyridazinon-Derivaten, wie sie in Anspruch 1, 2 oder 3 definiert sind, zur Bekämpfung von und/oder zum Schutz vor Schädlingen.

**9.** 2-Benzyl-5-hydroxy-3(2H)-pyridazinon.

**Revendications**

**Revendications pour les Etats contractants suivants : CH, DE, FR, GB, IT, LI**

**1.** Dérivé de pyridazinone de formule (I) :

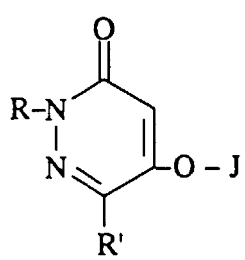

(I)

dans laquelle R représente un groupe alkyle ayant 1 à 4 atomes de carbone substitué par un groupe phényle,

R' représente un atome d'hydrogène,
J représente

$-CH_2-Q$, 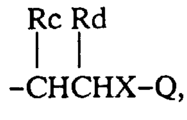, 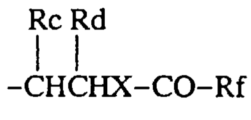 ou 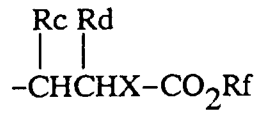 ;

où :

Rc et Rd représentent indépendamment un atome d'hydrogène ou un groupe alkyle ayant 1 à 4 atomes de carbone, Rf représente un atome d'hydrogène, un groupe alkyle ayant 1 à 4 atomes de carbone, un groupe alcényle ayant 2 à 8 atomes de carbone ou un groupe cycloalkyle ayant 3 atomes de carbone ;
X représente -O- ou -NH- ; et
Q représente un groupe pyridyle ou un groupe pyridyle substitué par un atome d'halogène.

**2.** Dérivé de pyridazinone de formule (I) selon la revendication 1

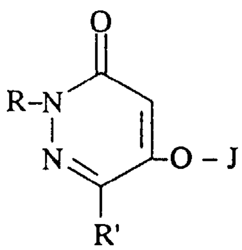

(I)

dans laquelle R représente un groupe alkyle ayant 1 atome de carbone substitué par un groupe phényle,

R' représente un atome d'hydrogène,
J représente

$-CH_2-Q$, 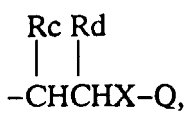, 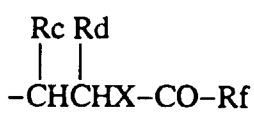 ou 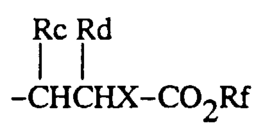 ;

où :

Rc et Rd représentent indépendamment un atome d'hydrogène ou un groupe méthyle, Rf représente un atome d'hydrogène, un groupe alkyle ayant 1 à 4 atomes de carbone, un groupe alcényle ayant 2 à 8 atomes de carbone ou un groupe cycloalkyle ayant 3 atomes de carbone ;
X représente -O- ou -NH- ; et
Q représente un groupe pyridyle ou un groupe pyridyle substitué par un atome d'halogène.

**3.** Dérivé de pyridazinone selon la revendication 1, comprenant les composés suivants :

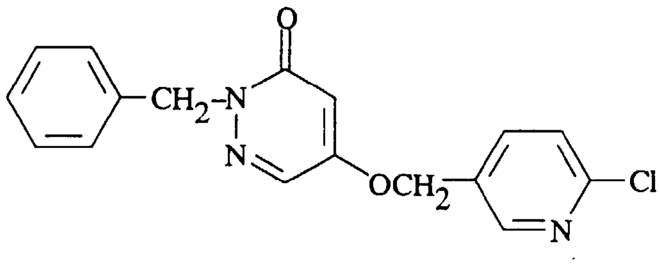

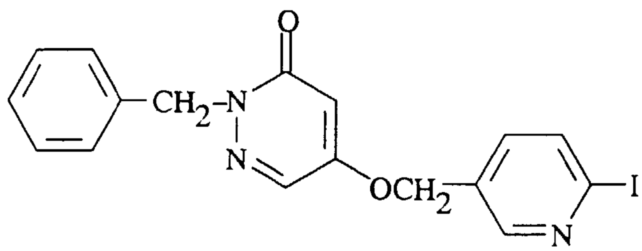

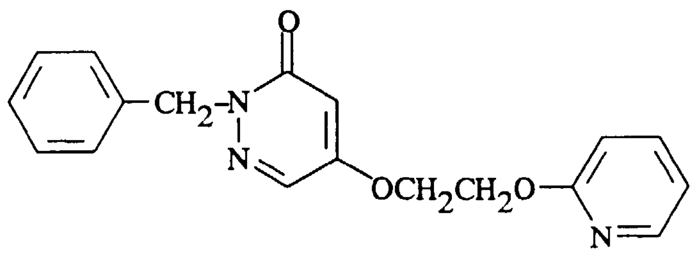

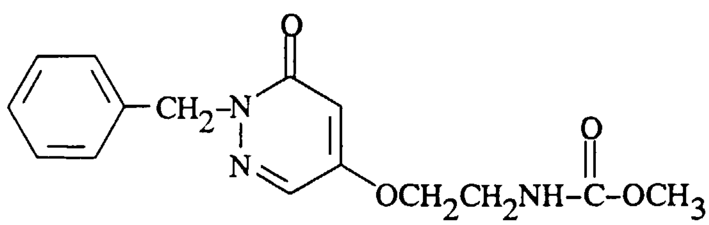

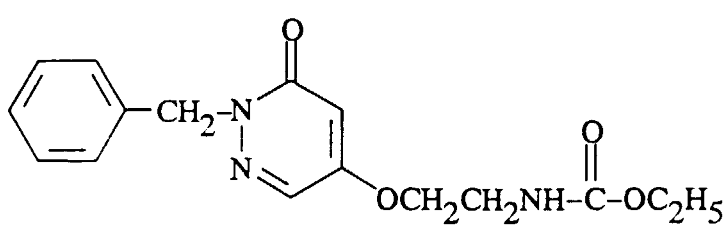

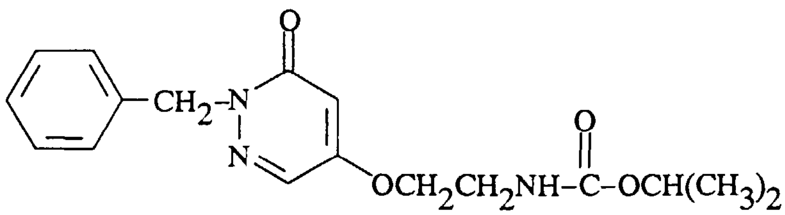

et

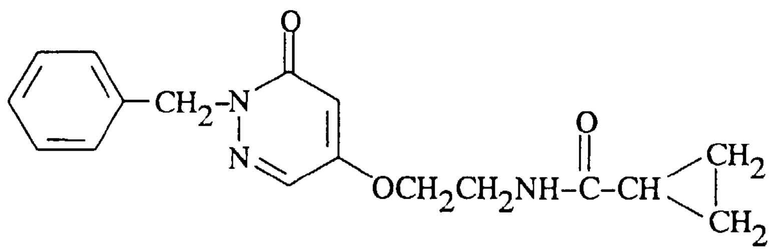

4. Procédé de préparation de dérivés de pyridazinone tels que définis dans la revendication 1 par réaction du composé de pyridazinone de formule générale (IB)

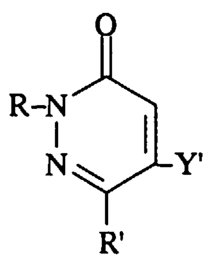

(IB)

avec le composé de formule générale (IC)

$$Y^2 - J \qquad (IC)$$

(où R, R' et J ont indépendamment la même signification que dans la revendication 1 ou 2, Y' et $Y^2$ représentent indépendamment un atome d'halogène ou un groupe OM (où M représente un atome d'hydrogène ou un métal alcalin), avec la condition que, lorsque Y' représente un atome d'halogène, $Y^2$ représente un groupe OM, et que, lorsque Y' représente un groupe OM, $Y^2$ représente un atome d'halogène).

5. Compositions pour la lutte et/ou la prévention contre les nuisibles contenant comme ingrédient actif un ou plusieurs des dérivés de 3(2H)-pyridazinone tels que définis dans la revendication 1.

6. Procédé non thérapeutique pour la lutte et/ou la prévention contre les nuisibles à un site qui comprend l'application audit site d'une quantité pesticide d'un ou plusieurs des dérivés de 3(2H)-pyridazinone tels que définis dans la revendication 1, en même temps qu'un support si nécessaire.

7. Procédé non thérapeutique selon la revendication 6, dans lequel les nuisibles contre lesquels il s'agit de lutter sont des insectes et sont représentatifs de l'ordre des acariens.

8. Utilisation non thérapeutique d'un dérivé de pyridazinone tel que défini dans la revendication 1, 2 ou 3 pour la lutte et/ou la prévention contre les nuisibles.

9. 2-benzyl-5-hydroxy-3-(2H)-pyridazinone.